# EUROPEAN PATENT APPLICATION

(11) **EP 1 749 485 A1**
(43) Date of publication of application: **07.02.2007**
(21) Application number: 06254005.9
(22) Date of filing: 31.07.2006
(51) Int. Cl.: A61B 17/072

(54) **Surgical instrument with an articulating shaft locking mechanism**

(30) Priority: 01.08.2005 US 194437
(71) Applicant: ETHICON ENDO-SURGERY, INC., Cincinnati, Ohio 45242 (US)
(72) Inventor: Wales, Kenneth S., Mason, Ohio 45040 (US); Boudeaux, Chad P., Cincinnati, Ohio 45242 (US)
(74) Representative: Tunstall, Christopher Stephen

(57) **Abstract**

A surgical instrument particularly suited to endoscopic use articulates an end effector by including a laterally sliding member in a proximal portion of a shaft that pivots the end effector to a selected side. Differentially opposing actuating forces (e.g., hydraulic, fluidic, mechanical) act against the laterally sliding member without binding by incorporating guidance mechanisms between the laterally sliding member and a frame of the shaft. A locking member advantageously unlocks automatically as articulation is commanded by resists backdriving of the mechanism.

## Description

### Cross Reference to Related Applications

The present invention claims the benefit of commonly owned U.S. Pat. Appln. 11/061,908 entitled "SURGICAL INSTRUMENT INCORPORATING A FLUID TRANSFER CONTROLLED ARTICULATION MECHANISM" to Kenneth Wales and Chad Boudreaux filed on February 18, 2005, the disclosure of which is hereby incorporated by reference in its entirety.
**Field of the Invention**

The present invention relates in general to surgical instruments that are suitable for endoscopically inserting an end effector (e.g., endocutter, grasper, cutter, staplers, clip applier, access device, drug/gene therapy delivery device, and an energy device using ultrasound, RF, laser, etc.) to a surgical site, and more particularly to such surgical instruments with an articulating shaft.
**Background of the Invention**

Endoscopic surgical instruments are often preferred over traditional open surgical devices since a smaller incision tends to reduce the post-operative recovery time and complications. Consequently, significant development has gone into a range of endoscopic surgical instruments that are suitable for precise placement of a distal end effector at a desired surgical site through a cannula of a trocar. These distal end effectors engage the tissue in a number of ways to achieve a diagnostic or therapeutic effect (e.g., endocutter, grasper, cutter, staplers, clip applier, access device, drug/gene therapy delivery device, and energy device using ultrasound, RF, laser, etc.).

Positioning the end effector is constrained by the trocar. Generally, these endoscopic surgical instruments include a long shaft between the end effector and a handle portion manipulated by the clinician. This long shaft enables insertion to a desired depth and rotation about the longitudinal axis of the shaft, thereby positioning the end effector to a degree. With judicious placement of the trocar and use of graspers, for instance, through another trocar, often this amount of positioning is sufficient. Surgical stapling and severing instruments, such as described in U.S. Pat. No. 5,465,895, are an example of an endoscopic surgical instrument that successfully positions an end effector by insertion and rotation.

Depending upon the nature of the operation, it may be desirable to further adjust the positioning of the end effector of an endoscopic surgical instrument. In particular, it is often desirable to orient the end effector at an axis transverse to the longitudinal axis of the shaft of the instrument. The transverse movement of the end effector relative to the instrument shaft is conventionally referred to as "articulation". This is typically accomplished by a pivot (or articulation) joint being placed in the extended shaft just proximal to the staple applying assembly. This allows the surgeon to articulate the staple applying assembly remotely to either side for better surgical placement of the staple lines and easier tissue manipulation and orientation. This articulated positioning permits the clinician to more easily engage tissue in some instances, such as behind an organ. In addition, articulated positioning advantageously allows an endoscope to be positioned behind the end effector without being blocked by the instrument shaft.

Approaches to articulating a surgical stapling and severing instrument tend to be complicated by integrating control of the articulation along with the control of closing the end effector to clamp tissue and fire the end effector (i.e., stapling and severing) within the small diameter constraints of an endoscopic instrument. Generally, the three control motions are all transferred through the shaft as longitudinal translations. For instance, U.S. Pat. No. 5,673,840 discloses an accordion-like articulation mechanism ("flex-neck") that is articulated by selectively drawing back one of two connecting rods through the implement shaft, each rod offset respectively on opposite sides of the shaft centerline. The connecting rods ratchet through a series of discrete positions.

Another example of longitudinal control of an articulation mechanism is U.S. Pat. No. 5,865,361 that includes an articulation link offset from a camming pivot such that pushing or pulling longitudinal translation of the articulation link effects articulation to a respective side. Similarly, U.S. Pat. No. 5,797,537 discloses a similar rod passing through the shaft to effect articulation.

In U.S. Pat. No. 5,673,841, certain deficiencies were recognized for then known articulating surgical instruments for endosurgical stapling, cutting, clip applying, and grasping. Specifically, when the surgical articulating instruments are loaded, the articulating head on the instrument tends to move. This movement is usually a combination of piece part deflection and slop (or backlash) in the articulation mechanism. High loads on the distal tip of the instrument (e.g., tissue clamping and staple firing) are reflected through the articulation device into the articulation control near the handle and can move (or rotate) the articulation control mechanism. In the past, articulation joints were designed with the articulation device performing double duty as the means for both positioning and locking the articulated head of the instrument. An examination of the force application points for the load (tip of the instrument) and the articulation device (near the articulation joint) reveals a mechanical disadvantage for the articulating device. This disadvantage manifests itself as a magnification of tolerances or clearances in the articulating device, resulting in significant head movements.

In response to this recognized deficiency, several locking mechanisms were proposed. In particular, a locking mechanism locks a head at an angle of articulation at all times except when it is desired to articulate the head with respect to the shaft. Upon actuation of the articulation device, for example by pulling an articulation band toward the proximal end of the instrument, the locking mechanism releases, unlocking the head and allowing articulation thereof. Discontinuation of the articulation step, for example, by stoppage of pulling forces on the articulation band, causes the locking mechanism to reengage, locking the head of the instrument in its new angle of articulation. In another version, a pair of fluid bladders on each side allow fluid flow to shift side to side to allow pivoting of the head with a pinch blade blocking the fluid flow to "lock" the articulating pivot.

While the articulation band effectively achieved articulation and simultaneous unlocking of the articulation joint, it is believed that in certain applications a direct linkage between control and the articulation joint may be desirable. Achieving proper dimensioning of bands without slippage or breakage may be deemed difficult. A degree of slop in tactile response given by the articulation control may also be undesirable.

Consequently, a significant need exists for an articulating joint of a surgical instrument that is directly linked to an articulation control that advantageously incorporates automatic locking of the articulation joint for resisting backdriving of the end effector.
**Brief Summary of the Invention**

The invention overcomes the above-noted and other deficiencies of the prior art by providing a surgical instrument whose elongate shaft pivotally articulates in response to an articulation linkage mechanism. Inadvertent change in this articulation angle and/or damage to the articulation linkage mechanism is avoided by an articulation joint lock that resists backloading of the end effector. Thereby, the articulation linkage mechanism may have a desirably small cross section.

In one aspect of the invention, a surgical instrument has an articulation control that a user actuates to cause pivoting of an end effector about an articulation joint of an elongate shaft. In particular, an articulation member extends a distal end from the elongate shaft into engagement with the end effector, being laterally deflected in response to the articulation control to effect articulation. In cooperation with this movement, a locking actuator is drawn proximally from the articulation joint, disengaging from an arcing braking surface attached to the end effector and radiating proximally about an articulation axis of the articulation joint. Thereby, a direct control of the articulation control is assisted by an articulation lock that maintains the end effector at a selected articulation angle without having to increase the size and strength of the articulation control to resist such backloading.

In another aspect of the invention, a surgical instrument incorporates an articulation joint that is controlled by lateral movement of a slide bar in an elongate shaft. A proximally directed gear segment attached to the end effector and aligned about an articulation axis of the articulation joint engages a distally directed rack on the slide bar. A locking member in the elongate shaft also translates longitudinally and distally into engagement with the gear segment to lock the articulation joint in position. Various advantages afforded by differentially moving a slide bar to effect articulation, such as various forms of motive power, are thus realized with the assurance of maintaining a desired articulation angle by means of the articulation lock.

In yet another aspect of the invention, a surgical instrument has an articulating shaft that is controlled by a slide bar received for lateral movement in the elongate shaft. A locking mechanism attached to the slide bar is advantageously moved into engagement with the elongate shaft in response to a backdriving force on the end effector.

These and other objects and advantages of the present invention shall be made apparent from the accompanying drawings and the description thereof.
**Brief Description of the Figures**

The accompanying drawings, which are incorporated in and constitute a part of this specification, illustrate embodiments of the invention, and, together with the general description of the invention given above and the detailed description of the embodiments given below, serve to explain the principles of the present invention.

FIGURE 1 is a front top perspective view of a surgical stapling and severing instrument shown with an open end effector, or staple applying assembly, and with the staple cartridge removed.

FIGURE 2 is a front top perspective view of the surgical stapling and severing instrument of FIG. 1 with an articulation mechanism actuated by a fluidic actuation control.

FIGURE 3 is a perspective disassembled view of an elongate shaft and articulation mechanism of the surgical stapling and severing instrument of FIG. 1.

FIGURE 4 is a perspective disassembled view of distal portions of an implement portion of the surgical stapling and severing instrument of FIG. 1, including the staple applying assembly and articulation mechanism.

FIGURE 5 is a top perspective view of the staple applying assembly of FIGS. 1 and 4 with a lateral half of a staple cartridge removed to expose components driven by a firing motion.

FIGURE 6 is a front perspective view of an implement portion of the surgical instrument of FIG. 1 with a double pivot closure sleeve assembly and end effector removed to expose a single pivot frame ground articulated by a fluidic articulation mechanism.

FIGURE 7 is perspective detail view of an alternative articulation joint for the surgical instrument of FIG. 1 depicting a double pivoting closure sleeve assembly at a proximal position with a single pivot frame ground.

FIGURE 8 is a bottom right perspective exploded view of the alternative articulation joint of FIG. 7 including a double pivoting fixed-wall dog bone link and a frame ground incorporating rail guides for a lateral moving member (T-bar).

FIGURE 9 is top left perspective exploded view of a further alternative articulation joint for the surgical instrument of FIG. 1, including an alternate solid wall support plate mechanism incorporated into a lower double pivot link to support a firing bar and includes a rail guided laterally moving member (T-bar).

FIGURE 10 is a top diagrammatic view of an alternate articulation locking mechanism for the surgical instrument of FIG. 1 with a closure sleeve assembly removed to expose a backloading disengaged T-bar for automatic articulation lock engagement and disengagement.

FIGURE 11 is a top diagrammatic view of an additional alternative articulation mechanism for the surgical instrument of FIG. 1, a spring biased rack on a T-bar with locking features that engage due to backloading from an end effector.

FIGURE 12 is an alternative T-bar and frame ground incorporating lateral guidance for the surgical instrument of FIG. 1.

FIGURE 13 is yet an additional alternative T-bar and frame ground incorporating lateral guidance for the surgical instrument of FIG. 1.

FIGURE 14 is a left top perspective disassembled view of an alternative articulation mechanism including a double pivoting frame assembly and single pivoting closure sleeve assembly for the surgical instrument of FIG. 1.

FIGURE 15 is a left bottom perspective view of the alternative articulation mechanism of FIG. 14.

FIGURE 16 is a diagram of a laterally moving fluidic articulation mechanism with rack and gear segment pivoting depicted in a nonarticulated state.

FIGURE 17 is cross section front view in elevation of the fluidic articulation mechanism of FIG. 16 taken along lines 17-17.

FIGURE 18 is a diagram of the laterally moving fluidic articulation mechanism with a rack and gear segment pivoting depicted in an articulated state.

FIGURE 19 is cross section front view in elevation of the fluidic articulation mechanism of FIG. 18 taken along lines 19-19.

FIGURE 20 is a top diagrammatic view of a surgical instrument articulated by at least one longitudinally moving member that laterally cams a slide bar, which in turn articulates an end effector.

FIGURE 21 is a top diagrammatic view of the surgical instrument of FIG. 20 in an articulated state.

FIGURE 22 is front cross section view in elevation of an alternative rotary link mechanical control system for a surgical instrument of FIGS. 16 or 20 for laterally translating respectively a T-bar or slide bar, depicted in an unarticulated state.

FIGURE 23 is a front cross section view in elevation of the alternative rotary link mechanical control system of FIG. 22 in an articulated state.

FIG. 24 depicts a perspective, exploded view of an alternative lateral articulation control mechanism for the alternative rotary link mechanical control system of FIG. 22.

FIG. 25 depicts a front elevation view in section of the lateral articulation control mechanism of FIG. 24.

FIG. 26 depicts a detail view of a locking block in an engaged state of the lateral articulation control mechanism of FIG. 24.

FIG. 27 depicts a detail view of the lateral articulation control mechanism of FIG. 24 in a disengaged state.

### Detailed Description of the Invention

### Overview of articulating shaft.

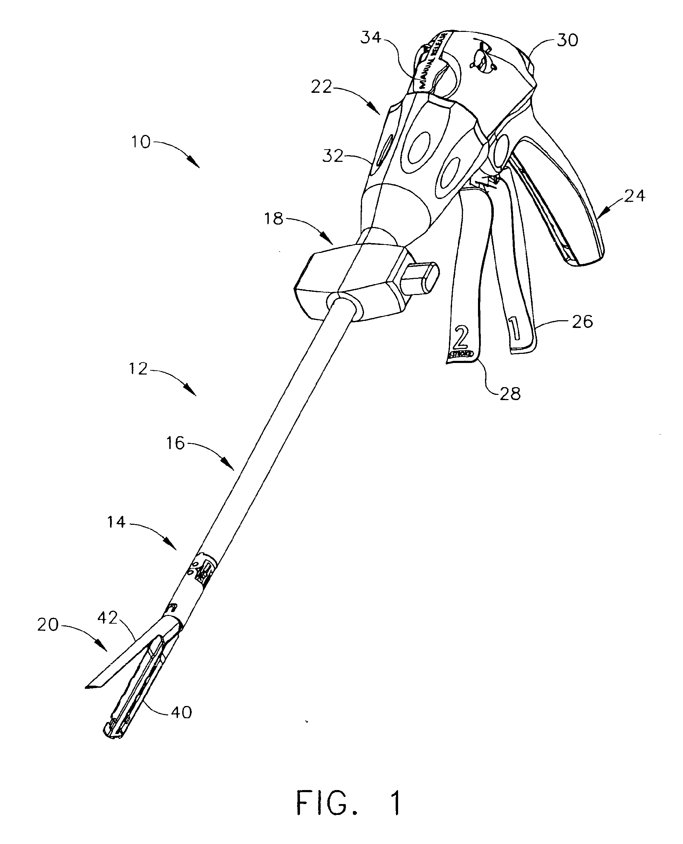

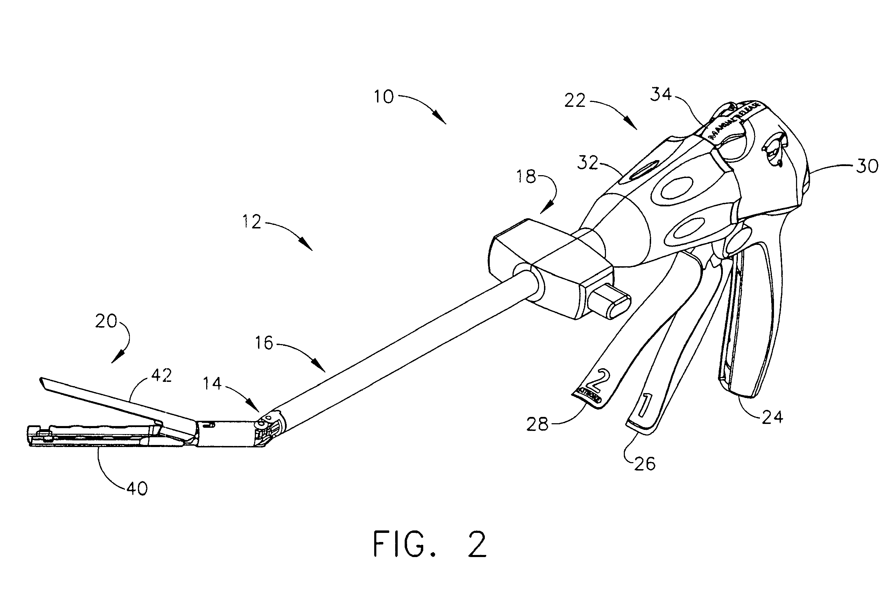

Turning to the Drawings, wherein like numerals denote like components throughout the several views, FIG. 1 depicts a surgical instrument, which in the illustrative versions is more particularly a surgical stapling and severing instrument 10, that is capable of practicing the unique benefits of the present invention. In particular, the surgical stapling and severing instrument 10 is sized for insertion, in a nonarticulated state as depicted in FIG. 1, through a trocar cannula passageway to a surgical site in a patient (not shown) for performing a surgical procedure. Once an implement portion 12 is inserted through a cannula passageway, an articulation mechanism 14 incorporated into a distal portion of an elongate shaft 16 of the implement portion 12 may be remotely articulated, as depicted in FIG. 2, by an articulation control 18. An end effector, depicted in the illustrative version as a staple applying assembly 20, is distally attached to the articulation mechanism 14. Thus, remotely articulating the articulation mechanism 14 thereby articulates the staple applying assembly 20 from a longitudinal axis of the elongate shaft 16. Such an angled position may have advantages in approaching tissue from a desired angle for severing and stapling, approaching tissue otherwise obstructed by other organs and tissue, and/or allowing an endoscope to be positioned behind and aligned with the staple applying assembly 20 for confirming placement.
Handle.

The surgical and stapling and severing instrument 10 includes a handle portion 22 proximally connected to the implement portion 12 for providing positioning, articulation, closure and firing motions thereto. The handle portion 22 includes a pistol grip 24 toward which a closure trigger 26 is pivotally and proximally drawn by the clinician to cause clamping, or closing, of the staple applying assembly 20. A firing trigger 28 is farther outboard of the closure trigger 26 and is pivotally drawn by the clinician to cause the stapling and severing of tissue clamped in the staple applying assembly 20. Thereafter, a closure release button 30 is depressed to release the clamped closure trigger 26, and thus the severed and stapled ends of the clamped tissue. The handle portion 22 also includes a rotation knob 32 coupled for movement with the elongate shaft 16 to rotate the shaft 16 and the articulated staple applying assembly 20 about the longitudinal axis of the shaft 16. The handle portion 22 also includes a firing retraction handle 34 to assist in retracting a firing mechanism (not depicted in FIGS. 1-2) should binding occur, so that opening of the staple applying assembly 20 may occur thereafter.

It will be appreciated that the terms "proximal" and "distal" are used herein with reference to a clinician gripping a handle of an instrument. Thus, the surgical stapling assembly 20 is distal with respect to the more proximal handle portion 22. It will be further appreciated that for convenience and clarity, spatial terms such as "vertical" and "horizontal" are used herein with respect to the drawings. However, surgical instruments are used in many orientations and positions, and these terms are not intended to be limiting and absolute.

An illustrative multi-stroke handle portion 22 for the surgical stapling and severing instrument 10 of FIGS. 1-2 is described in greater detail in the co-pending and commonly-owned U.S. patent application entitled "SURGICAL STAPLING INSTRUMENT INCORPORATING A MULTISTROKE FIRING POSITION INDICATOR AND RETRACTION MECHANISM" to Swayze and Shelton IV, Ser. No. 10/674,026, the disclosure of which is hereby incorporated by reference in its entirety, with additional features and variation as described herein. While a multi-stroke handle portion 22 advantageously supports applications with high firing forces over a long distance, applications consistent with the present invention may incorporate a single firing stroke, such as described in co-pending and commonly owned U.S. patent application "SURGICAL STAPLING INSTRUMENT HAVING SEPARATE DISTINCT CLOSING AND FIRING SYSTEMS" to Frederick E. Shelton IV, Michael E. Setser, and Brian J. Hemmelgarn, Ser. No. 10/441,632, the disclosure of which is hereby incorporated by reference in its entirety.
Implement Portion (Articulating Elongate Shaft And Staple Applying Assembly).

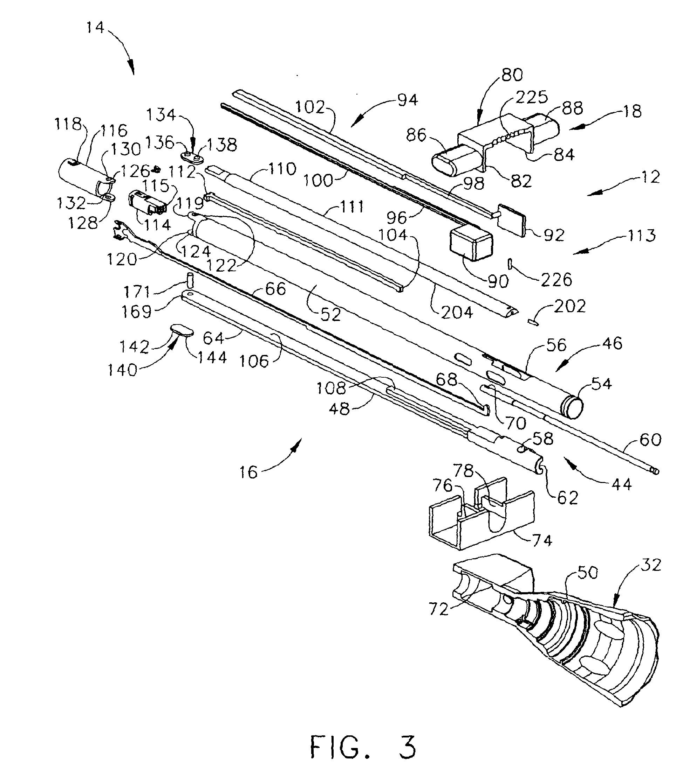

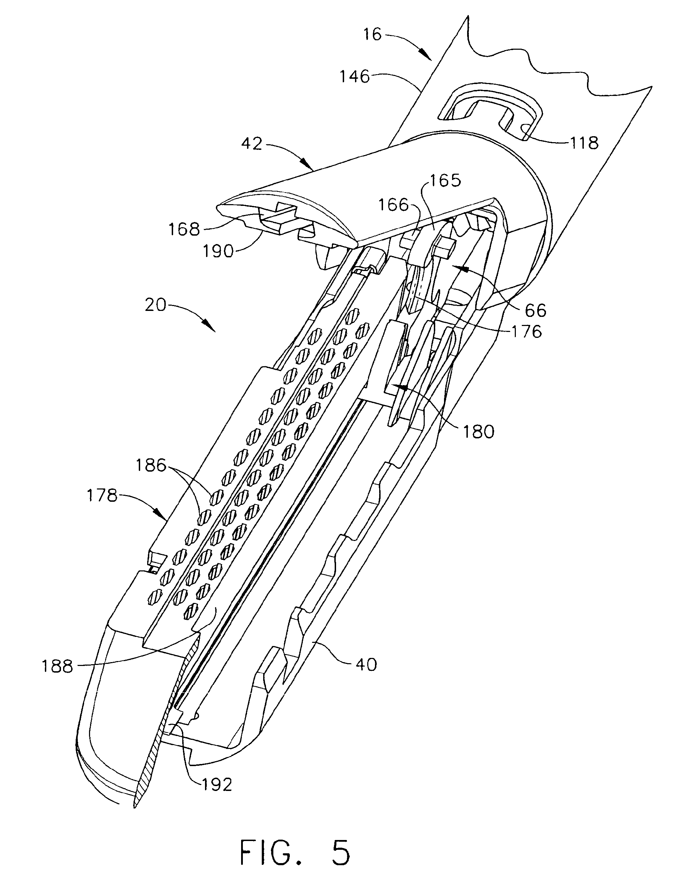

In FIGS. 1-5, the implement portion 12 advantageously incorporates the multiple actuation motions of longitudinal rotation, articulation, closure and firing within a small diameter suitable for endoscopic and laparoscopic procedures. The staple applying assembly 20 ("end effector") has a pair of pivotally opposed jaws, depicted as an elongate channel 40 with a pivotally attached anvil 42 (FIGS. 1-2, 4-5). Closure and clamping of the anvil 42 to the elongate channel 40 is achieved by longitudinally supporting the elongate channel 40 with a frame assembly 44 (FIG. 3) rotatingly attached to the handle portion 22 over which a double pivot closure sleeve assembly 46 longitudinally moves to impart a closing and opening respectively to a distal and proximal motion to the anvil 42, even with the staple applying assembly 20 articulated as in FIG. 2.

With particular reference to FIG. 3, the frame assembly 44 includes a single pivot frame ground 48 whose proximal end is engaged to the rotation knob 32, with a right half shell 50 thereof shown in FIG. 3. It should be appreciated that a proximal end of the closure sleeve assembly 46, specifically of a closure straight tube 52, with one end encompassing the proximal end of the frame ground 48 and the other end passing into the handle portion 22, engages closure components (not shown) that longitudinally translate the closure sleeve assembly 46. A circular lip 54 at the proximal end of the closure straight tube 52 provides a rotating engagement to such components. Engaging components of the rotation knob 32 pass through a longitudinal slot 56 on a proximal portion of the straight closure tube 52 to engage an aperture 58 which is proximally positioned on the frame ground 48. The longitudinal slot 56 is of sufficient length to allow the longitudinal closure translation of the closure sleeve assembly 46 even at various rotational angles set by the rotation knob 32 of the closure sleeve assembly 46 and the frame ground 48.

The elongate shaft 16 supports the firing motion by receiving a firing rod 60 that rotatingly engages firing components of the handle portion 22 (not shown). The firing rod 60 enters a proximal opening 62 along the longitudinal centerline of the frame ground 48. The distal portion of the frame ground 48 includes a firing bar slot 64 along its bottom that communicates with the proximal opening 62. A firing bar 66 longitudinally translates in the firing bar slot 64 and includes an upwardly projecting proximal pin 68 that engages a distal end 70 of the firing rod 60.

The elongate shaft 16 supports articulation by incorporating a rectangular reservoir cavity 72, one lateral portion depicted in a distal portion of the rotation knob 32. A bottom compartment 74 that resides within the rectangular reservoir cavity 72 has laterally spaced apart left and right baffles 76, 78. An articulation actuator 80 slides laterally overtop of the bottom compartment 74, its downward laterally spaced left and right flanges 82, 84, which are outboard of the baffles 76, 78, each communicating laterally to left and right push buttons 86, 88 that extend outwardly from the respective shell halves of the rotation knob 32. The lateral movement of the articulation actuator 80 draws left and right flanges 82, 84 nearer and farther respectively to the left and right baffles 76, 78, operating against left and right reservoir bladders 90, 92 of a fluidic articulation system 94, each reservoir bladder 90, 92 communicating respectively and distally to left and right fluid conduits or passageways 96, 98 that in turn communicate respectively with left and right actuating bladders 100, 102. The latter oppose and laterally pivot a T-bar 104 of the articulation mechanism 14.

The frame assembly 44 constrains these fluidic actuations by including a top and distal recessed table 106 of the frame ground 48 upon which resides the fluid passages 96, 98 and actuating bladders 100, 102. The T-bar 104 also slidingly resides upon the recessed table 106 between the actuating bladders 100, 102. Proximal to the T-bar 104, a raised barrier rib 108 is aligned thereto, serving to prevent inward expansion of the fluid passages 96, 98. The frame assembly 44 has a rounded top frame cover (spacer) 110 that slides overtop of the frame ground 48, preventing vertical expansion of the fluid passages 96, 98 and actuating bladders 100, 102, as well as constraining any vertical movement of the T-bar 104. In particular, the frame cover 110 includes features that enable it to also provide an articulation locking member 111, described in greater detail below as part of an articulation locking mechanism 113.

A distal end ("rack") 112 of the T-bar 104 engages to pivot a proximally directed gear segment 115 of an articulated distal frame member 114 of the articulation mechanism 14. An articulated closure tube 116 encompasses the articulated distal frame member 114 and includes a horseshoe aperture 118 that engages the anvil 42. A double pivoting attachment is formed between the closure straight tube 52 and articulating closure ring 116 over the articulating mechanism 14, allowing longitudinal closure motion even when the articulating mechanism 14 is articulated. In particular, top and bottom distally projecting pivot tabs 118, 120 on the closure straight tube 52 having pin holes 122, 124 respectively are longitudinally spaced away from corresponding top and bottom proximally projecting pivot tabs 126, 128 on the articulating closure ring 116 having pin holes 130, 132 respectively. An upper double pivot link 134 has longitudinally spaced upwardly directed distal and aft pins 136, 138 that engage pin holes 130, 122 respectively and a lower double pivot link 140 has longitudinally spaced downwardly projecting distal and aft pins 142, 144 that engage pin holes 132, 124 respectively. A vertical pin hole 169 distally positioned through the frame ground 48 receives a frame pivot pin 171 that pivots in an underside of the distal frame member 114.

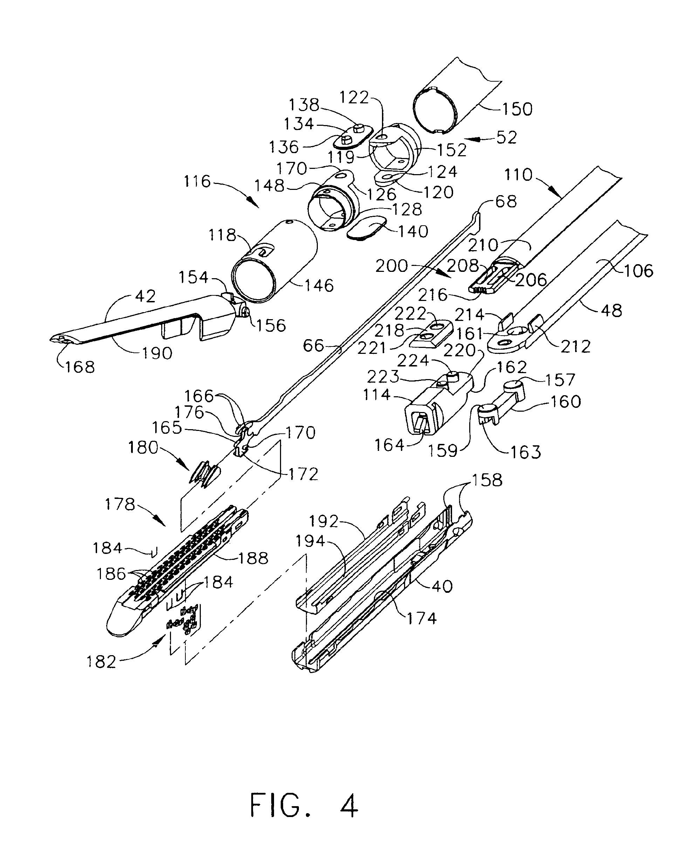

With particular reference to FIG. 4, the articulating closure ring 116 is shown for enhanced manufacturability to include a short tube 146 attached to an articulating attachment collar 148 that includes the proximally projecting pivot tabs 126, 128. Similarly, the straight closure tube 52 is assembled from a long closure tube 150 that attaches to an aft attachment collar 152 that includes the distally projecting pivot tabs 119, 120. The horseshoe aperture 118 in the short closure tube 146 engages an upwardly projecting anvil feature 154 slightly proximal to lateral pivot pins 156 that engage pivot recesses 158 inside of the elongate channel 40.

The alternative version of FIG. 4 includes a dog bone link 160 instead of a frame pivot pin 171 whose proximal pin 157 pivotally attaches to the frame ground 48 in a frame hole 161 and whose distal pin 159 rigidly attaches to a proximal undersurface 162 of the articulating distal frame member 114, thereby providing pivotal support there between. A bottom longitudinal knife slot 163 in the dog bone link 160 guides an articulating portion of the firing bar 66. The articulating distal frame member 114 also includes a bottom longitudinal slot 164 for guiding a distal portion of the firing bar 66.
Staple Applying Apparatus (End Effector).

With reference to FIGS 4-5, the firing bar 66 distally terminates in an E-beam 165 that includes upper guide pins 166 that enter an anvil slot 168 in the anvil 42 to verify and assist in maintaining the anvil 42 in a closed state during staple formation and severing. Spacing between the elongate channel 40 and anvil 42 is further maintained by the E-beam 165 by having middle pins 170 slide along the top surface of the elongate channel 40 while a bottom foot 172 opposingly slides along the undersurface of the elongate channel 40, guided by a longitudinal opening 174 in the elongate channel 40. A distally presented cutting surface 176 of the E-beam 165, which is between the upper guide pins 166 and middle pins 170, severs clamped tissue while the E-beam 165 actuates a replaceable staple cartridge 178 by distally moving a wedge sled 180 that causes staple drivers 182 to cam upwardly driving staples 184 out of upwardly open staple holes 186 in a staple cartridge body 188, forming against a staple forming undersurface 190 of the anvil 42. A staple cartridge tray 192 encompasses from the bottom the other components of the staple cartridge 178 to hold them in place. The staple cartridge tray 192 includes a rearwardly open slot 194 that overlies the longitudinal opening 174 in the elongate channel 40, thus the middle pins 170 pass inside of the staple cartridge tray 192.

The staple applying assembly 20 is described in greater detail in co-pending and commonly-owned U.S. Patent Application Ser. No. 10/955,042, "ARTICULATING SURGICAL STAPLING INSTRUMENT INCORPORATING A TWO-PIECE E-BEAM FIRING MECHANISM" to Frederick E. Shelton IV, et al., filed 30 September 2004, the disclosure of which is hereby incorporated by reference in its entirety.
Articulation Locking Mechanism.

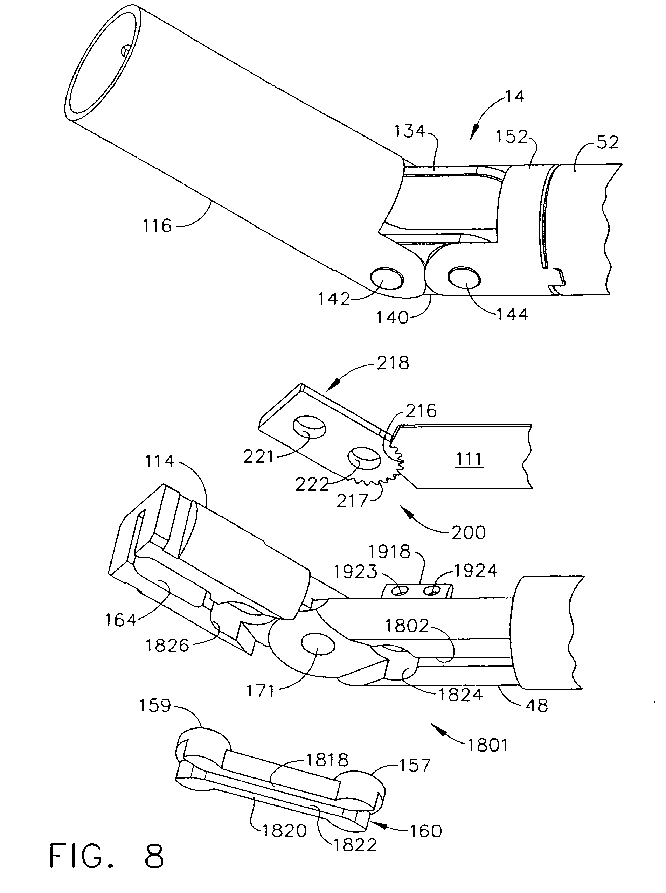

In FIGS. 3-4, and 6-8, an articulation lock mechanism 200 is advantageously incorporated to maintain the staple applying assembly 20 at a desired articulation angle. The articulation lock mechanism 200 reduces back driven loads on the left and right actuating bladders 100, 102. In particular, a compression spring 202 (FIG. 3) is proximally positioned between a proximal end 204 of the articulation locking member 111 and the handle portion 22, biasing the articulation locking member 111 distally. With particular reference to FIG. 4, two parallel slots 206, 208 at a distal end 210 of the articulation locking member 111 receive respectively upwardly projecting guide ribs 212, 214 on the frame ground 48. The guide ribs 212, 214 are longitudinally shorter than the parallel slots 206, 208 allowing a range of relative longitudinal travel. Thereby, with particular reference to FIG. 8, selective abutting engagement of a distal frictional surface, depicted as a toothed recess 216 distally projecting from the articulation locking member 111 is engaged to a corresponding locking gear segment 217 in a brake plate 218 received into a top proximal recess 220 (FIG. 4) of the articulating frame member 114. Distal and proximal holes 221, 222 in the brake plate 218 receive distal and proximal pins 223, 224 that upwardly project from the top proximal recess 220.

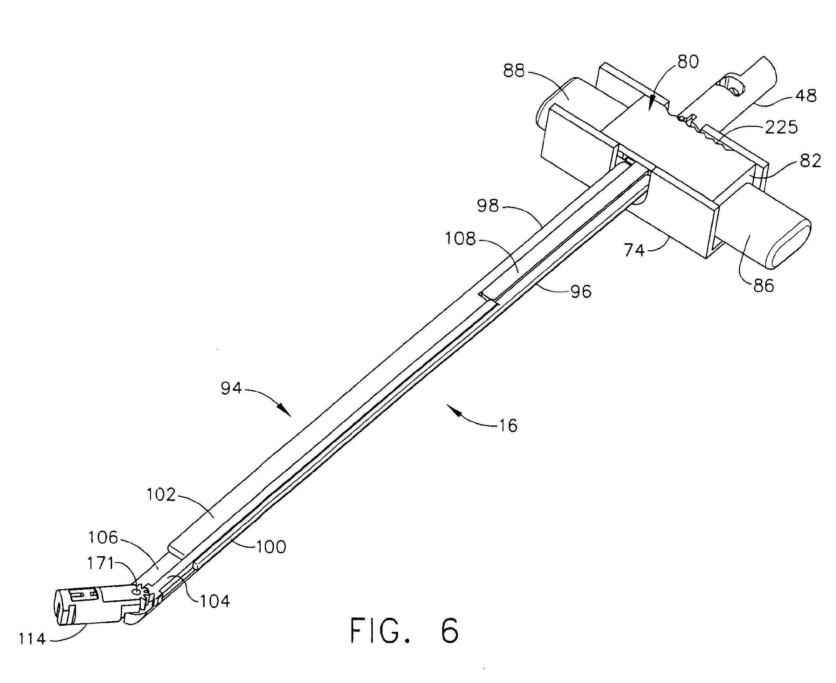

With particular reference to FIG. 6, the elongate shaft 16 is depicted in an articulated position with the closure sleeve assembly 46 removed from around the frame assembly 44 and without the elongate channel 40 and anvil 42. Articulation actuator 80 is shown moved laterally to the left to compress right proximal reservoir bladder 92 and expand distal right actuation bladder 102 moving T-bar 104 to the position shown. Thus, lateral movement of the articulation actuator 80 articulates the distal frame 114 clockwise about the single pivot frame ground 48 as viewed from above. The articulation actuator 80 advantageously also automatically engages and disengages the articulation lock mechanism 200. In particular, a toothed detent surface 225 along a proximal top surface of the articulation actuator 80 receives a downwardly projecting locking pin 226 from the proximal end 204 of the articulation locking member 111 (not shown in FIG. 6). The engagement of the locking pin 226 within the root of the toothed detent surface 225 provides sufficient distal movement of the articulation locking member 111 for locking engagement of the locking gear segment 217 in the brake plate 218. Lateral movement by an operator of the articulation member 80 proximally urges the locking pin 226 proximally, and thus disengages the articulation locking member 111 from the brake plate 218. When the operator releases the articulation actuator 80, the locking pin 226 is urged by the compression spring 202 into the adjacent detent in detent surface 225 to lock the locking mechanism 111, and thereby the staple applying assembly 20, constraining the articulation mechanism 14 at a desired articulation position by constraining and expanding the inflated shape of the proximal left and right reservoir bladders 90, 92.

Alternatively or additionally, an orifice may be provided within parallel fluid conduits 96, 98 to control the flow rate between the distal actuating bladders 100, 102 and proximal reservoir bladders 90, 92 .

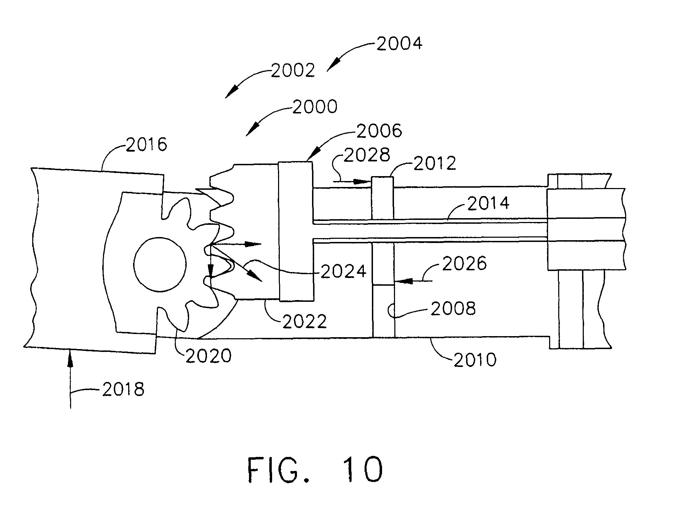

In FIG. 10, an alternate locking mechanism 2000 of an articulation mechanism 2002 of a surgical instrument 2004, is normally unlocked and is activated by cocking a laterally moving T-bar 2006 due to back loading. A slot 2008 is located in a frame ground 2010 to receive and guide a rib 2012 extending down from the T-bar 2006. A slender longitudinal section 2014, which is orthogonally attached to the rib 2012 deflects if an end effector 2016 is backloaded. For instance, as the end effector 2016 is forced to the right as depicted at arrow 2018, for instance, its proximal gear segment 2020 acts upon a rack 2022 of the T-bar 2006, imparting a nonorthogonal backdriving force, as depicted at arrow 2024. Thus, the slender longitudinal section 2014 bends, cocking rib 2012 in slot 2008. This cocking produces opposing binding forces, as depicted by arrows 2026, 2028, that lock the T-bar 2006 and prevent further articulation. Unlocking occurs when actuation of the articulation bladders uncocks the laterally moving T-bar 2006. Thereafter, the rib 2012 may assist in guiding the T-bar 2006.

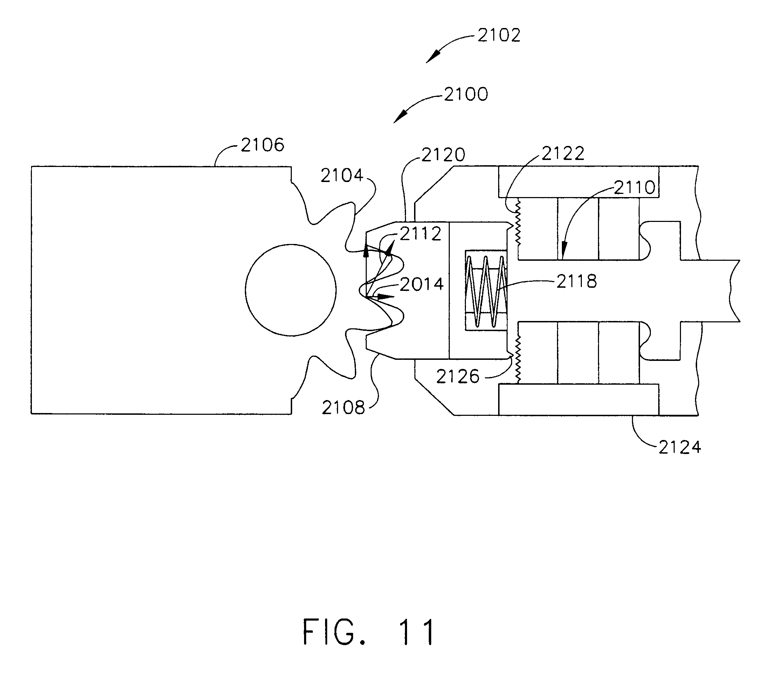

In FIG. 11, yet an additional articulation locking mechanism 2100 for a surgical instrument 2102 is depicted that is normally unlocked and activated by the proximal force vector from the twenty (20) degree pressure angle from gear teeth 2104 of an end effector 2106 and rack teeth 2108 of a T-bar 2110. When the end effector 2106 is backloaded, as depicted by nonorthogonal arrow 2112, the longitudinal vector of the pressure angle, depicted as arrow 2114, moves the T-bar 2110 proximally. This longitudinal force vector is applied to a stiff spring 2118 behind a rack 2120 of the T-bar 2110. When the spring 2118 deflects as T-bar 2110 moves proximally, locking teeth 2126 projecting proximally from the rack 2120 are brought into engagement with locking elements 2122 distally projecting and laterally aligned on a ground frame 2124. The locking teeth 2126 and locking elements 2122 disengage when the proximal force vector 2014 is reduced or eliminated by removing the back loading of the end effector 2106 and allowing T-bar 2110 to move distally from urging from spring 2118.
Double Pivot Closure Sleeve and Single Pivot Frame Ground Combination.

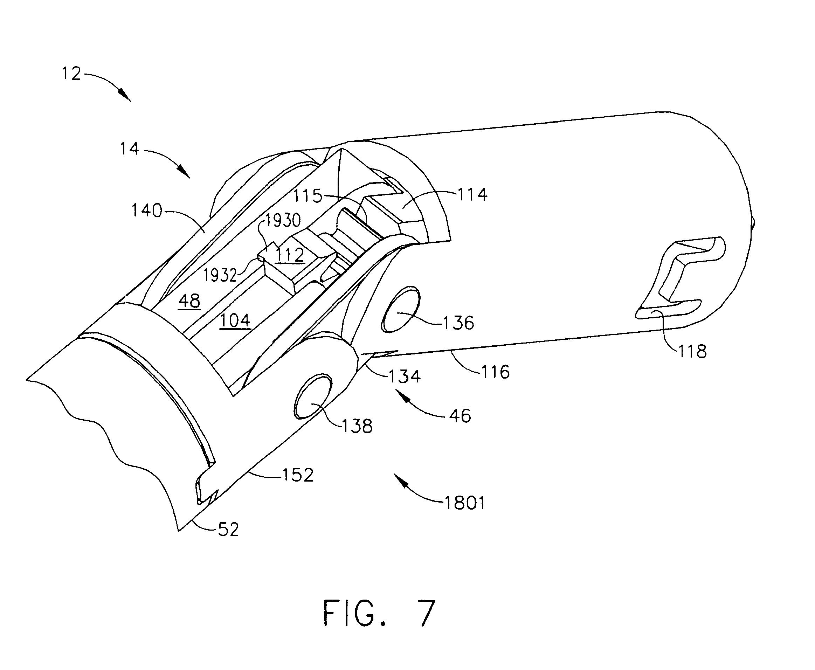

With reference to FIGS. 3-4 and 7, the implement portion 12 advantageously incorporates the double pivot closure sleeve assembly 46 that longitudinally translates over and encompasses a single pivot frame ground 48. These mechanisms and their operation will now be described in further detail. With particular reference to FIG. 7, the articulation mechanism 14 is depicted in an articulated state with the closure sleeve assembly 46 retracted proximally to an anvil open state. With the anvil 42 open (not shown in FIG. 7), actuation of the articulation control 18 causes the articulated closure ring 116 to pivot about the upwardly directed distal pin 136 and downwardly directed distal pin 142 (not shown in FIG. 7) respectively of the upper and lower double pivot closure links 134, 140. The frame ground 48 pivots around a single pin, depicted as the frame pivot pin 171 (FIG. 3) that joins frame ground 48 to distal frame member 114. With the anvil 42 open, the frame pivot pin 171 of frame ground 48 is aligned with the distal most position of upper and lower double pivot links 134, 140 of the closure sleeve assembly 46. This positioning allows easy pivoting and rotation of the staple applying assembly 20 while the anvil 42 is open. When the closure sleeve assembly 46 is moved distally to pivot anvil 42 closed, the closure straight tube 52 moves distally about frame ground 48 and the articulated closure ring 116 moves distally along the articulated distal frame member 114 axis as urged by pivot links 134, 140. Dual pivoting pins 136, 138 and 142, 144 on links 134, 140 facilitate engagement with closure straight tube 52 and articulated closure ring 116 as they are urged towards the distal closure position when the device is articulated (not shown). At the distal closure position, the frame pivot pin 171 is vertically aligned with proximal pivot pins 138, 144 at full articulation or may fall at any point between distal pins 136, 142 and proximal pins 138, 144 while working effectively.
Solid Firing Bar Support.

In FIG. 8, the articulation mechanism 14 of FIG. 7 is partially exploded and viewed from the bottom, showing a solid wall firing bar support design (dog bone link 160) of FIG. 4 that offers advantages over conventional flexible support plates. Support plates are used to bridge the gap and guide and support the firing bar 66 through a single frame ground pivot articulation joint 1801. Flexible firing bars are known, but the incorporation of solid wall firing bars such as those shown in FIGS. 4, 8 and 9 offer unique advantages. Referring now to FIG. 8, frame ground 48 includes a frame knife slot 1802 that runs along the bottom of frame ground 48 and a distal knife slot 164 runs along the bottom of the articulating distal frame member 114 for the sliding reception of the firing bar 66 (not shown) therein. Frame ground 48 is described above and includes a direct single pivotal connection at frame pivot pin 171 with the distal frame member 114. The fixed wall dog bone link 160 that is rotatably connected on proximal pin end 157 and movably connected on distal pin end 159 includes left and right lateral guides 1818, 1820, defining therebetween a guidance slot 1822 for sliding passage of a firing bar 66 (FIG. 4).

Thus, to bridge the gap between frame ground 48 and the distal frame member 114, the fixed wall pivoting dog bone link 160 is pivotally attached to frame ground 48 and is slidingly attached to frame member 114. Proximal pin 157 of the pivoting dog bone 160 is pivotally received in a bore 1824 in frame ground 48 enabling pivotal dog bone 160 to pivot about pocket 1824. A distal pin 159 extends upwards from pivotal dog bone 160 and is slidingly received in a slot 1826 in distal frame member 114. Articulation of staple applying assembly 20 to an angle such as 45 degrees from the longitudinal axis pivots pivoting dog bone 116 in bore 1824 at its proximal pin 157 and distal pin 159 slides into slot 1826 formed in the distal frame member 114 to bend firing bar 66 to two spaced-apart angles that are half of the angle of the staple applying assembly 20. Unlike previously referenced flexible support plates that bend the firing bar 66 to a 45 degree angle, the fixed wall pivoting dog bone 160 bends the firing bar 66 to two spaced-apart angles such as 22.5 degrees each. Bending the flexible firing bar or bars 66 to half the angle cuts the bend stress in the firing bars 66 to one-half of that found in conventional articulation supports. Reducing the bending stress in the firing bars 66 reduces the possibility of permanently bending or placing a set in the firing bars, reduces the possibility of firing jams, ensures lower firing bar retraction forces, and provides smoother operation of the firing system.

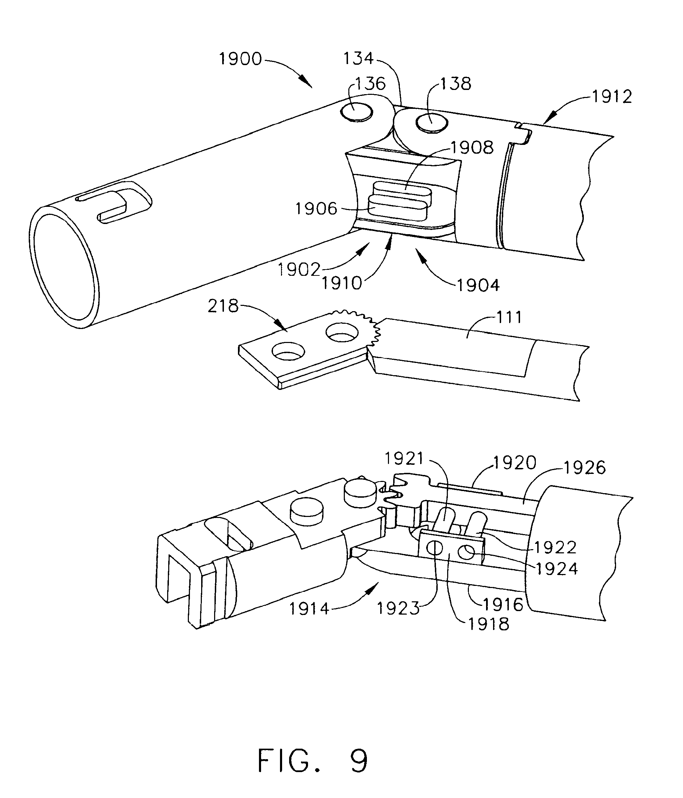

In FIG. 9, a surgical instrument 1900 includes double closure pivot. Single frame pivot articulation joint 1902 shows an alternate solid wall support plate mechanism 1904 that replaces the lower double pivot link 140 and dog bone link 1812 of FIG. 8. Left and right firing bar supports 1906, 1908 extend upwardly from a lower double pivot link 1910 of a closure sleeve assembly 1912. Clearance 1914 is provided in a frame ground 1916 for the firing bar supports 1906, 1908 to travel as the closure sleeve assembly 1912 moves distally to close the anvil 42 (not shown in FIG. 9) and proximally to open anvil 42. Like the above described pivoting dog bone 160, the alternate lower double pivoting link 1910 also bends and supports the firing bar 66 (not shown in FIG. 9) creating two spaced apart bend angles that are up to one-half of the bend angle of the staple applying assembly 20.
Lateral Member Guide Mechanisms.

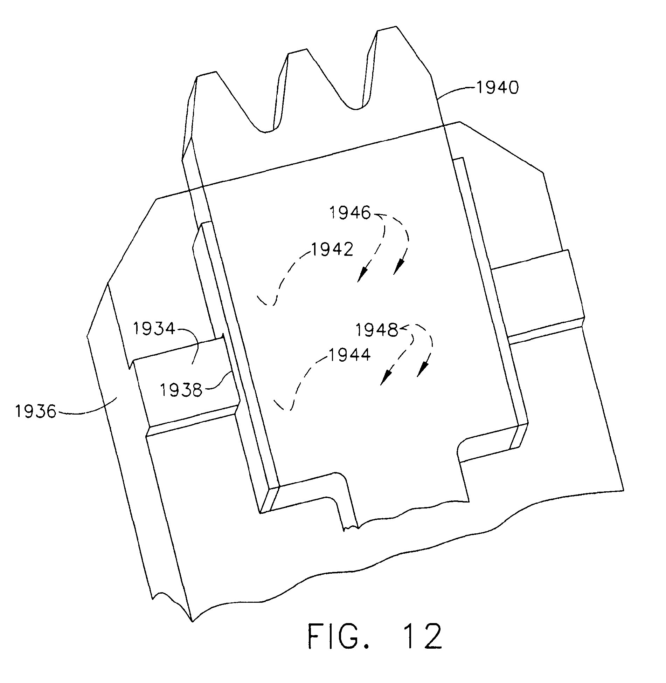

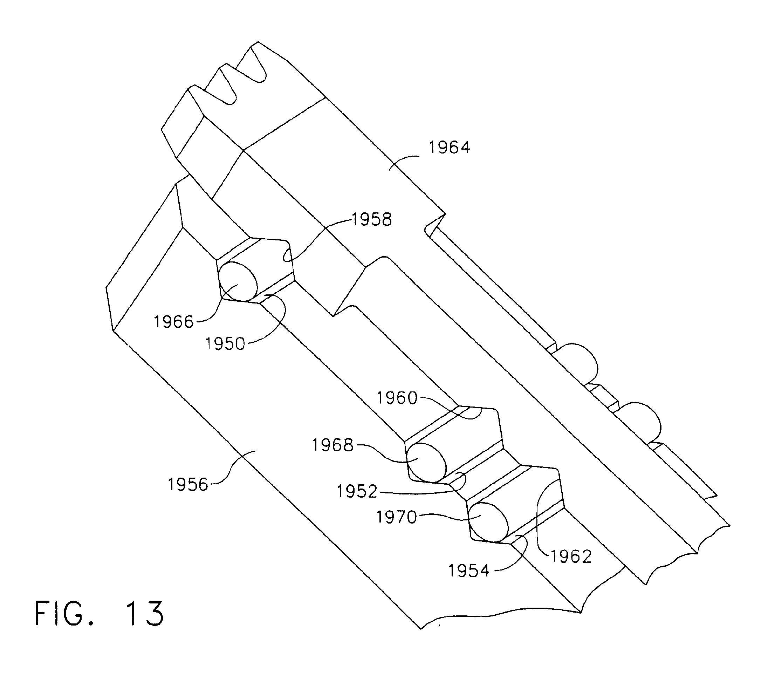

With further reference to FIG. 9, left and right upward flanges 1918, 1920 on the frame ground 1916 include distal and proximal lateral pin guides 1921, 1922, 1923, 1924 that pass laterally through holes 1923, 1924 in a T-bar 1926 assisting in minimizing binding in an articulation mechanism 1928. These pin guides 1921, 1922 are also incorporated into the frame ground 48 of FIG. 7. As another example, in FIG. 7, the T-bar 104 advantageously includes a dovetail lateral guide 1930 that laterally slides within a dovetail channel 1932 formed in the frame ground 48. As yet a further example, in FIG. 12, a raised rib 1934 on a frame ground 1936 is received within a rectangular slot 1938 formed in a T-bar 1940. To further facilitate non-binding lateral translation, distal and proximal lateral bearing tracks 1942, 1944 each include a respective plurality of ball bearings 1946, 1948. As yet a further example, in FIG. 13, a plurality of frame lateral grooves 1950-1954 are formed in a frame ground 1956 with corresponding T-bar lateral grooves 1958-1962 in a T-bar 1964. Slide rollers 1966-1970 reside trapped within respective pairs of lateral grooves 1950/1958, 1952/1960, 1954/1962. These are by no means an exhaustive list of lateral guidance members that prevent unwanted cocking or rotation of the T-bar 1964.
Double Pivot Frame Ground and Single Pivot Closure Combination.

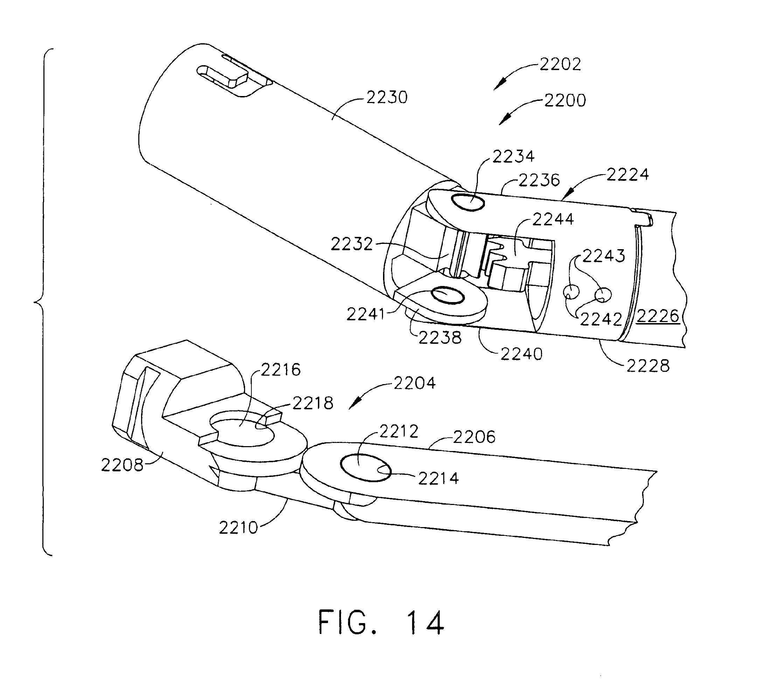

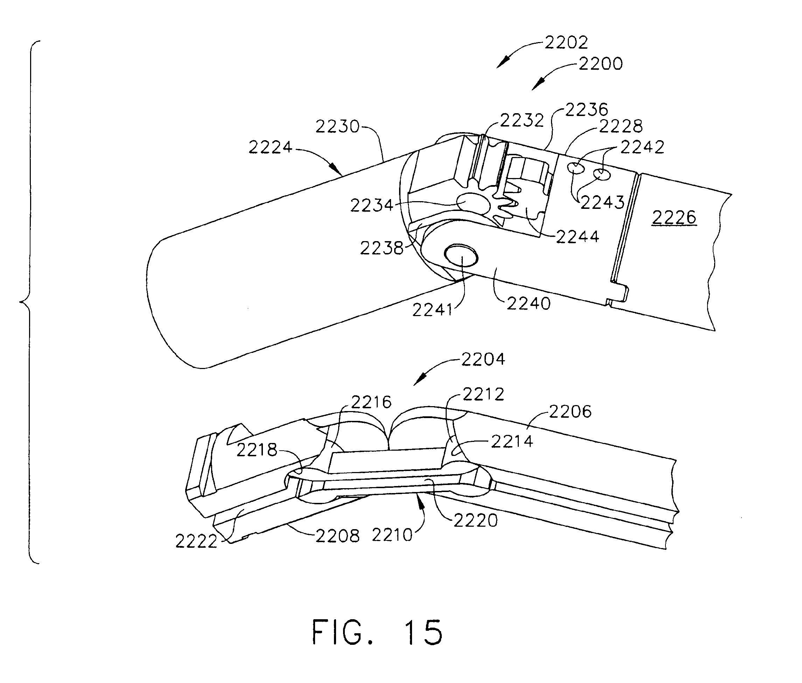

In FIGS. 14-15, an alternate frame ground and closure mechanism 2200 is incorporated into a surgical instrument 2202 that includes double pivoting frame assembly 2204. In particular, a frame ground 2206 is connected to distal frame member 2208 by a dual pivot frame dog bone 2210 having a proximal pivot pin 2212 pivotally engaging a proximal bore 2214 in frame ground 2206 and a distal pivot pin 2216 engaging a distal bore 2218 of distal frame member 2208. A guidance slot 2220 is located on the underside of dog bone 2210 for the guidance of a firing bar 66 (not shown in FIGS. 14-15) therein. Knife slot 2222 is located in distal frame member 2208. As shown, articulation of a closure ring 2230 of a closure sleeve assembly 2224 to a forty-five (45) degree angle articulates distal frame member 2208 to a forty-five (45) degree angle and articulates frame dog bone 2210 to half that angle. Consequently, firing bar 66 is subjected to the two shallow half bends that are spaced apart and obtains all the benefits listed above.

Outermost closure sleeve assembly 2224 is different in that only one pivot axis of the double pivoting design of the frame assembly 2204 accommodates its longitudinal closure motion. As shown, a closure tube shaft 2226 has a clevis 2228 at a distal end. Clevis 2228 is pivotally engaged with the closure ring 2230. Closure ring 2230 has a proximal gear 2232 formed at a distal end. A pin 2234 passes through the proximal gear 2232 and pivotally engages an upper tang 2236 of clevis 2228. A lower arm 2238 is pivotally engaged to a lower tang 2240 of clevis 2228 by an aligned pin 2241. Holes 2242 in the clevis 2228 receive lateral guides pins 2243 and slidably attach a T-bar 2244 therein to engage proximal gear 2232 of the closure ring 2230. Thus, this alternate mechanism 2200 uses a reversed single/dual pivot alternate concept from the previously described mechanism. That is, the alternate closure mechanism 2200 has a single pivot and the alternate frame ground has a dual pivot, unlike the previously described dual pivot closure mechanism with a single pivot frame ground.
Laterally Moving Articulation Mechanism

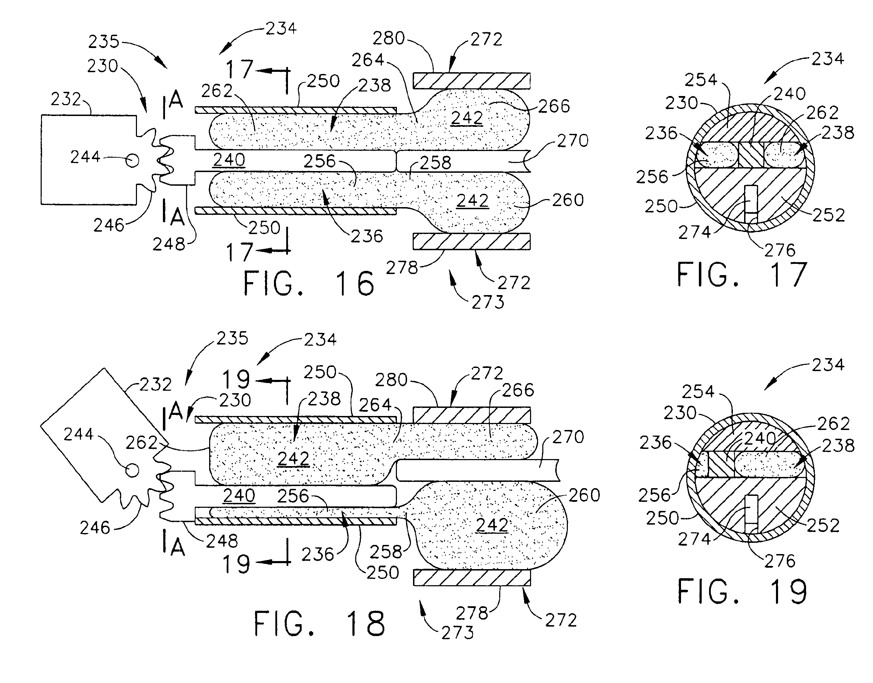

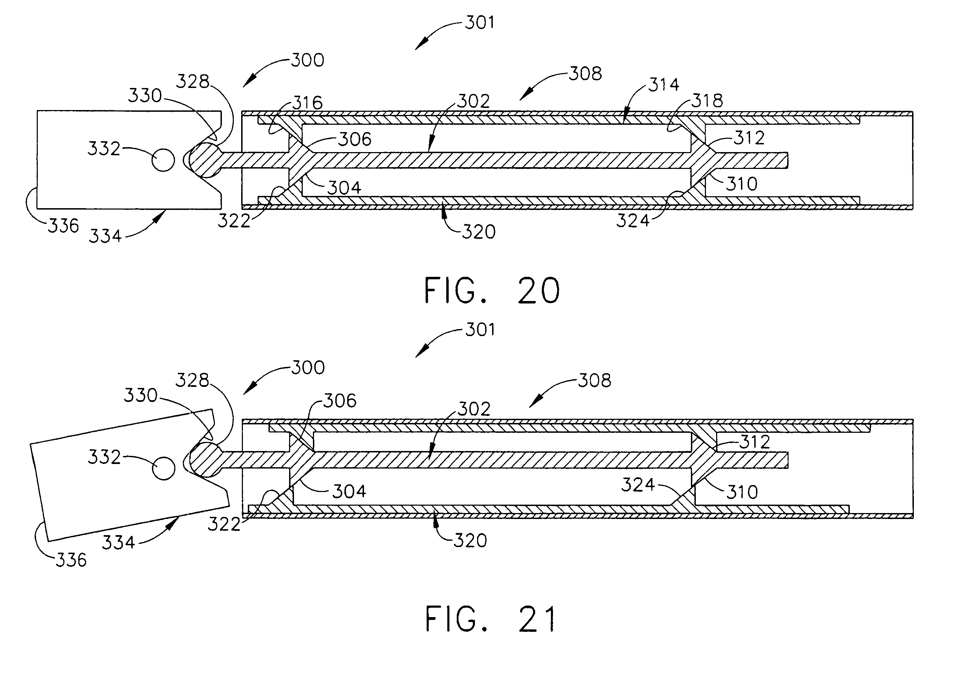

In FIGS. 16-19, a laterally moving articulation mechanism 230 is depicted schematically to show lateral motion being used to effect articulation of an end effector 232. Lateral motion is the movement of at least one element toward or away from the longitudinal axis of a surgical device 234. This motion is generally at right angles to the longitudinal axis, which is a horizontal line bisecting the mechanism 230, and does not involve rotational motion or longitudinal motion. Laterally moving articulation mechanisms can be fluid actuated as shown in FIGS. 16-19 or mechanically actuated as shown in FIGS. 20-23.
Laterally Moving Fluid Articulation Mechanism

The laterally moving articulation mechanism 230 is shown schematically in FIGS. 16-19 and includes a fluid control system 235 having fluid-filled parallel left and right fluid bladders 236, 238 extending longitudinally therein that move a lateral member or T-bar 240 laterally by the movement of fluids 242. All directions are in reference to the longitudinal axis. Referring to the unarticulated view of FIGS. 16 and 17, the distally located end effector 232 pivots about pin 244 and has a gear segment 246 at a proximal end. Pivot pin 244 is attached to a frame (not shown). A rack 248 at a distal end of the T-bar 240 operably engages gear segment 246. T-bar 240 and rack 248 are laterally moveable along axis A-A. Respective distal portions of the long left and right fluid bladders 236, 238 lie laterally to the laterally moveable T-bar 240 and are laterally constrained within a closure sleeve 250 and vertically constrained by a frame 252 below and a spacer 254 above. In particular, left actuating fluid bladder 236 has left distal actuating bladder 256, left fluid passageway 258, and a left proximal reservoir bladder 260. Right fluid bladder 238 has a right distal actuating bladder 262, right fluid passageway 264, and right proximal reservoir bladder 266. A fixed divider 270 extends from the frame 252 and separates the bladders 260, 266 and the fluid passageways 258, 264. The fixed divider 270 and the closure sleeve 250 constrain the fluid passageways 258, 264 and prevent expansion in the fluid passage sections 258, 264 of the bladders 236, 238. A laterally moveable "C" shaped compression member 272 is included in articulation control mechanism 273 for the compression of one of the proximal reservoir bladders 260, 266 and the articulation of the end effector 232. In addition, other components such as a firing bar 274 passing through a firing bar slot 276 in the frame 252 may be incorporated (FIGS. 17, 19).

As shown in FIGS. 8-19, lateral movement of C-shaped compression member 272 to the left compresses right proximal reservoir bladder 266 forcing fluid 242 into right fluid passageway 264 and right distal actuating bladder 262. As right distal actuating bladder 262 moves T-bar 240 laterally to the left, the left distal actuating bladder 256 is compressed and the end effector 232 is articulated to the right (clockwise as viewed from the top as shown). Compression of the left distal actuating bladder 256 causes fluid 242 to flow proximally through the left fixed fluid passageway 258 and into left proximal reservoir bladder 260. In particular, an attached right wall 280 of the C shaped compression member 272 moves to the left causing compression of the right proximal reservoir bladder 266. A corresponding movement left of an attached left wall 278 of the C shaped compression member 272 provides space for the fluid from compressed left actuator bladder 256 as the fluid flows into the expanding left proximal reservoir bladder 260.

This fluid control system 235 for the articulation mechanism 230 offers at least several advantages. First, the orientation of the actuating bladders 256, 262, proximal to the articulation joint or mechanism 230, allows the use of long bladders 236, 238 and longer T-bars 240 within the surgical device 234. As a fluid-driven system, increasing the output force of the fluid control system 235 may be accomplished in two ways. First, for a fixed fluid area on the T-bar 240, the fluid pressure onto the fixed area may be increased. Second, for a fixed fluid pressure, the fluid contact area on the T-bar 240 may be increased. The first method results in a more compact design and higher system pressures. The second method results in a larger design and lower system pressures. To decrease cost, simplify the design, reduce system stress, and reduce risk of bladder rupture, the illustrative version depicts long distal actuating bladders 256, 262 in an advantageous position proximal to the articulation mechanism 230 within an elongate shaft of the surgical device 234. It is this placement of the bladders 256, 262 that enable the bladders 256, 262 to be long and the articulation output force to be high for a low input pressure.

Thus, the output force of the articulation mechanism 230 can be increased (for the same input pressure) simply by increasing the pressure contact area of the distal actuating bladders (balloons) 256, 262 on T-bar 240. Pressure contact area increases are restricted to height and length. Since the diameter of conventional endoscopic surgical instruments are fixed at certain diameters to pass through insufflation ports, this limits the height change. Changing the length of the pressure contact area has the greatest effect and enables the lateral output force of the device to be advantageously tuned (by changing length) to meet whatever output force the system requires.

Fluids used in a laterally moving device can be either compressible or incompressible. As used herein, the term "fluid" comprises liquids, gases, gels, microparticles, and any other material which can be made to flow between a pressure gradient. While any fluid can be used, sterilized solutions such as saline, mineral oil or silicone are especially preferred.
Laterally Moving Mechanical Articulation Mechanism

Whereas fluid mechanisms are described above to cause lateral movement and articulation, mechanical mechanisms may accomplish a similar lateral motion as produced by fluid bladders 236, 238. In FIGS. 20-21, an alternate laterally moving articulation mechanism 300 employs a mechanical control system, in particular a longitudinally moving member, to affect lateral motion and articulation for a surgical instrument 301. In the illustrative version, with particular reference to FIG. 20, a laterally moving slide bar 302 has at least one pair of angled left and right cam surfaces 304, 306 extending laterally therefrom on opposite sides of an elongate longitudinal shaft 308. In the illustrative version, another pair of proximal left and right angled cam surfaces 310, 312 are also included. A right longitudinally moving link 314 includes corresponding inwardly directed distal and proximal counter ramped surfaces 316, 318 that register and slidingly engage to distal and proximal right cam surfaces 306, 312 such that distal longitudinal movement of the moving link 314 causes leftward lateral movement of the slide bar 302. It should be appreciated that this ramping contact may be reversed such that distal movement causes rightward movement respectively.

It should be appreciated that a spring bias (not shown) may be included on the slide bar 302 to urge the slide bar 302 rightward into engagement with the right longitudinally moving link 314 so that the opposite proximal movement of the right longitudinal moving link 314 allows leftward movement of the slide bar 302. Alternatively, in the illustrative version, a left longitudinally moving link 320 includes corresponding inwardly directed distal and proximal counter ramped surfaces 322, 324 that register and slidingly engage to distal and proximal right cam surfaces 304, 310, the latter ramp distally and the former ramp proximally so that distal longitudinal movement of the left longitudinally moving link 320 causes rightward lateral movement of the slide bar 302. It should be appreciated that this ramping contact may be reversed such that proximal movement causes leftward movement. It should be appreciated that the right and left longitudinally moving links 314, 320 and sliding bar 302 are supported within the elongate shaft 308 that allows this longitudinal movement of the former and lateral movement of the latter.

A distal end of the slide bar 302, depicted as a socket ball 328, is received within a V-shaped cam groove 330 proximally aligned and proximal to a pivot pin 332 of an end effector 334. Thus, in FIG. 21, proximal movement of the right longitudinally moving link 314 and distal movement of the left longitudinally moving link 320 causes rightward movement of the sliding bar 302 with a corresponding rightward movement of the socket ball 328. Thus the V-shaped cam groove 330 is driven rightward, pivoting its most distal end 336 to the left. Alternatively, lateral movement of the slide bar 302 may be converted to articulation of the end effector 334 by the rack and gear engagement described above with respect to FIGS. 16-19. Thus, mechanical systems that use longitudinal movement can be used to provide lateral articulation for the surgical instrument 301.
Rotatable Link.

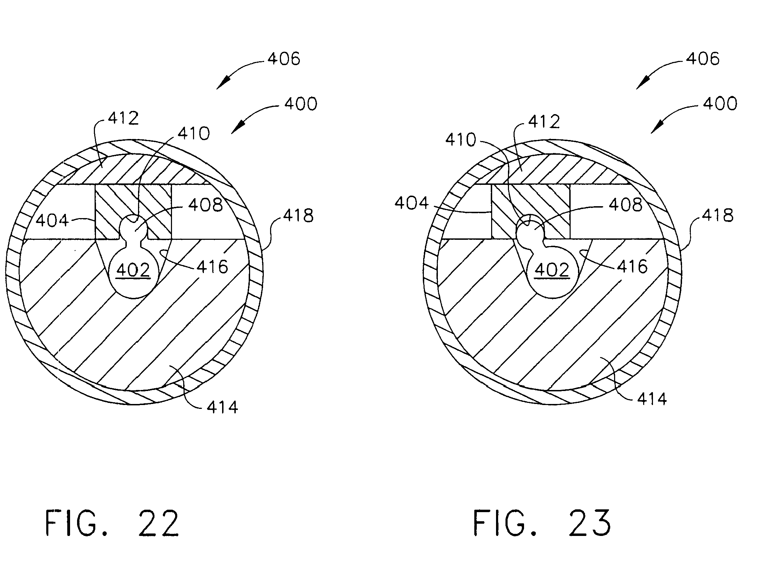

In FIGS. 22 and 23, a further alternate articulation mechanism 400 uses a rotatable link 402 to move a lateral member, depicted as laterally moving slide bar 404, to cause articulation for a surgical instrument 406. The laterally moving slide bar 404 may operably engage with a rotary gear or a cammed groove as described above for FIGS. 16 and 20 at a proximal end of an end effector (not shown). Rotatable link 402 may be located below the slide bar 404 with at least one arm 408 extending rotatably transverse to the longitudinal axis therefrom to engage within a socket 410 within the slide bar 404. The slide bar 404 is vertically constrained between a top spacer 412 and a bottom frame 414, the later having a longitudinal trough 416 that receives the rotatable link 402 and accommodates rotation of the arm 408. The spacer 412 and frame 414 are encompassed by a tubular sleeve 418. Rotation of the rotary link 402 moves the arm 408 in an arc and thereby moves the slide bar 404 laterally in the direction of rotation.
Lateral-to-Rotary One-Way Control Actuator.

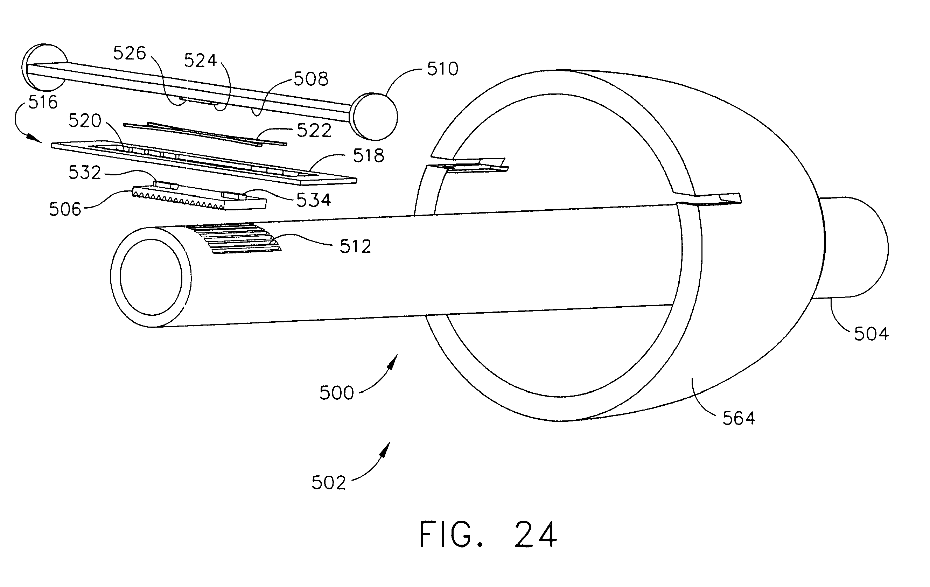

In FIGS. 24-27. it is desirable to provide an automatic locking feature that resists backdriving of the rotatable link 402. To that end, the rotatable link 402 is coupled to a lateral articulation control 500 that was described for use with different articulation joints in a co-pending and commonly-owned U.S. Pat. Appln. No. 10/615,972 entitled "SURGICAL INSTRUMENT WITH A LATERAL-MOVING ARTICULATION CONTROL", the disclosure of which is hereby incorporated in its entirety. The lateral articulation control 500 may be adapted for use in the articulation control 18 for an alternative articulating surgical instrument 502 similar to that described for FIGS. 1-6. In particular, the lateral articulation control 500 converts a lateral motion into a rotational motion transferred by an articulation drive tube 504 to an articulation mechanism (not shown in FIGS. 24-27). Adapting this to the previously mentioned articulation control 18 may entail acting as a one-way clutch between two laterally moving surfaces. Returning to FIGS. 24-27, a downward projecting gear rack 506 is coupled to a lower side 508 of a lateral control actuator 510 for engaging with longitudinally aligned grooves 512 on a top face of the articulation drive tube 504.

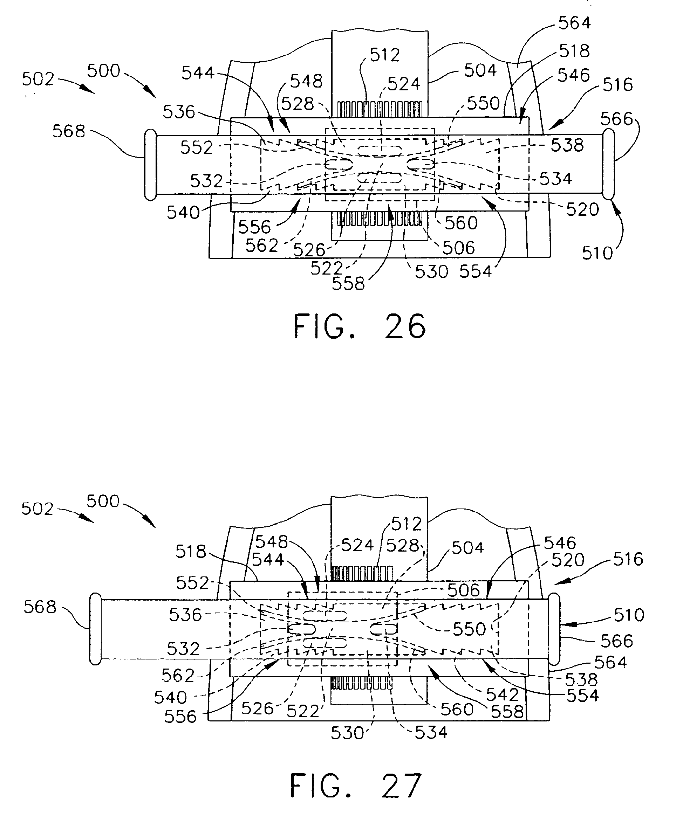

An articulation backdrive lockout 516 is advantageously incorporated into the lateral articulation control 500 to prevent a force upon the end effector (not depicted in FIGS. 24-27) from changing the amount of articulation. In particular, interposed between the articulation control actuator 510 and the gear rack 506 is a rack plate 518 that includes a central opening 520 containing a flexible X-shaped locking member 522. The articulation control actuator 510 includes two deflection blades 524, 526 that downwardly project into the central opening 520 of the rack plate 518 and are positioned respectively in a distal and a proximal quadrant defined by the X-shaped locking member 522 with respect to a top view depicted in FIGS. 26-27. The gear rack 506 includes two drive blades 532, 534 that upwardly project into the central opening 520 of the rack plate 518 and are positioned respectively in the left and right quadrants 536, 538 defined by the X-shaped locking member 522. The central opening 520 of the rack plate 518 is shown as being generally rectangular in shape, but with ramped teeth 540, each presenting an abutting surface 542 inwardly facing and longitudinally aligned. These ramped teeth 540 are placed along a right and left portion 544, 546 of a distal edge 548 to ratchedly contact right and left distal arms 550, 552 respectively of the X-shaped locking member 522. The ramped teeth 540 are also placed along a right and left portion 554, 556 of a proximal edge 558 of the rectangular window 520 to ratchedly contact right and left proximal arms 560, 562 of the X-shaped locking member 522.

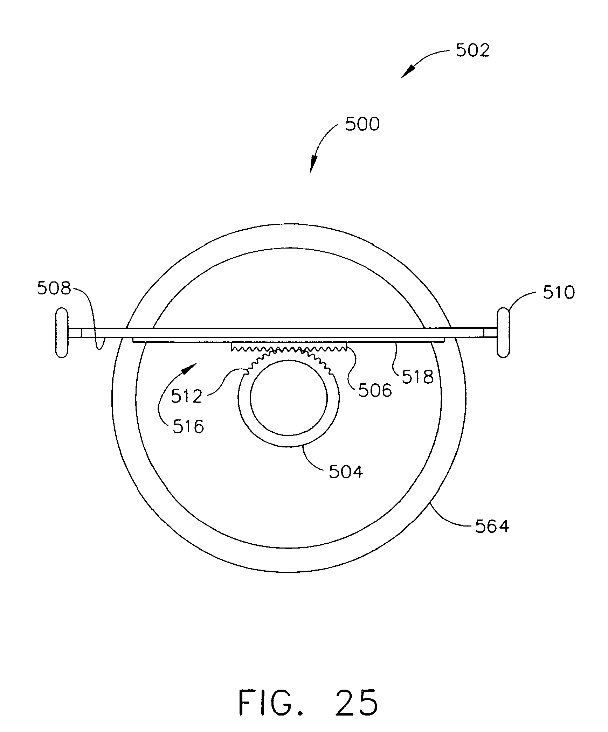

With particular reference to FIG. 25, the gear rack 518 is illustrated as attached to a knob 564 and thus does not laterally translate with the articulation control actuator 510 or the gear rack 506. Lateral movement of the articulation control actuator 510 is transferred through the articulation backdrive lockout 516 formed inside the rectangular window 520 of the rack frame 518. By contrast, a backdriven lateral movement of the articulation drive tube 504 and hence the gear rack 506 is reacted by the articulation backdrive lockout 516 into the rack frame 518 and into the knob 560. Thus movement of the articulation drive tube 504 is arrested.

In use, as depicted in FIG. 26, the lateral articulation control 500 is centered. Thereby, a visual indication is given to the clinician by the equally extended right and left ends 566, 568 of the articulation control actuator 510. The deflection blades 524, 526 are centered on the X-shaped lockout member 522, exerting no force on the arms 550, 552, 560, 562, which are thereby allowed to extend toward their uncompressed state into abutting contact with the ramped teeth 540, preventing lateral movement of the X-shaped lockout member 522. The drive blades 532, 534 of the gear rack 506 are in opposing contact on each side of the X-shaped lockout member 522. Any lateral force transferred from the articulation drive tube 504 into the gear rack 506 through the drive blades 532, 534 is reacted through the X-shaped lockout member 522 into the gear rack 506, preventing movement.

By contrast, as depicted in FIG. 27, when a clinician moves the articulation control actuator 510 to one lateral side, the deflection blades 524, 526 contact a pair of proximal and distal arms (the left ones 552, 562 in FIG. 27) compressing the pair away from contact with the rectangular window 520. Thus, the X-shaped lockout member 522 is allowed to move in that direction with the trailing pair of arms (e.g., right ones 550, 560 in FIG. 27) ratcheting along. This lateral movement is allowed to continue until the leading arms 552, 562 encounter the lateral extent of the rectangular window 520 as depicted. The drive blades 532, 534 of the gear rack 506 move with the X-shaped lockout member 522 and thus ultimately the end effector (not shown in FIG. 27) also articulates in response.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For instance, a single fluid transfer approach may be incorporated wherein a single fluid actuator expands and compresses to effect articulation, perhaps assisted by a resilient opposing member that is not in fluid or pneumatic communication to the handle. An application consistent with such a design, for instance, could include just one bladder attached to a T-bar so that when compressed by the withdrawal of fluid, it pulls the T-bar with it.

## Claims

1. A surgical instrument, comprising:
a proximal portion configured for manipulation external to a patient;
an elongate shaft attached to the proximal portion;
an end effector;
an articulation joint pivotally attaching the end effector to the elongate shaft;
an arcing braking surface attached to the end effector and radiating proximally about an articulation axis of the articulation joint;
an articulation control attached to the proximal portion;
an articulation member extending a distal end from the elongate shaft into engagement with the end effector, the distal end laterally deflected in response to the articulation control to effect articulation;
a locking actuator guided by the elongate shaft, proximally coupled to the articulation control, distally terminating in a locking surface positioned to selectively engage the locking surface.

2. The surgical instrument of claim 1, wherein the articulation control comprises a lateral control operatively configured to proximally urge the locking actuator out of engagement with the end effector during movement of the lateral control.

3. The surgical instrument of claim 2, wherein the locking member is distally biased and wherein the engagement surface comprises a proximal pin, and the lateral gear segment comprises a toothed surface that cams the pin proximally during actuation allowing the proximal pin to distally move into a corresponding tooth root of the toothed surface when the articulation control stops.

4. The surgical instrument of claim 2, wherein the lateral control comprises a differential fluid control.

5. The surgical instrument of claim 1, further comprising:
a slide bar constrained for lateral movement within the elongate shaft;
a distal end of the slide bar positioned in the articulation joint; and
a proximal surface of the end effector engaged to the distal end of the articulation movement for converting a lateral motion of the slide bar to a pivoting motion of the end effector.

6. The surgical instrument of claim 1, wherein the elongate shaft further comprises an articulation drive tube transferring a rotational motion to the articulation joint to effect pivoting articulation of the end effector, and wherein the locking actuator comprises a backdrive lockout mechanism interposed between the articulation drive tube and the articulation control.

7. The surgical instrument of claim 6, wherein the backdrive lockout mechanism comprises:
a frame having a window;
a lockout member laterally locked into position with the window of the frame and coupled to the lateral gear rack; and
a deflection member coupled to the articulation control and positioned to disengage and to laterally position the lockout member.

8. The surgical instrument of claim 6, wherein the articulation drive tube comprises a rotatable link aligned parallel to the slide bar and aligned with a vertical centerline of the elongate shaft, the rotatable link pivotally joined to a near surface of the slide bar causing lateral movement thereof as the rotatable link rotates.

9. The surgical instrument of claim 8, wherein the rotatable link includes a plurality of pivotal joinings to the slide bar spaced along a longitudinal length thereof to maintain the alignment during actuation.

10. A surgical instrument, comprising:
a proximal portion configured for manipulation external to a patient;
an elongate shaft attached to the proximal portion;
an end effector;
an articulation joint pivotally attaching the end effector to the elongate shaft;
a gear segment proximally directed and attached to the end effector aligned to rotate about an articulation axis of the articulation joint;
a slide bar constrained for lateral movement within the elongate shaft;
a distally directed rack attached to the slide bar positioned in the articulation joint in engagement with the gear segment of the end effector;
a proximal surface of the end effector engaged to the distal end of the articulation movement for converting a lateral motion of the slide bar to a pivoting motion of the end effector; and
a locking member in the elongate shaft selectively, distally and longitudinally translating to engage the gear segment of the end effector locking articulation joint.

11. The surgical instrument of claim 10, wherein the locking member is distally biased and includes a proximal pin, the articulation control including a toothed surface positioned to cam the pin proximally during actuation and to allow the proximal pin to distally move into a corresponding tooth root of the toothed surface when the articulation control stops.

12. The surgical instrument of claim 10, further comprising differential articulation actuators positioned on opposing sides of the slide bar.

13. The surgical instrument of claim 12, wherein the articulation actuators comprise fluidic actuators.

14. The surgical instrument of claim 12, wherein the articulation actuators comprise electromagnetic actuators.

15. The surgical instrument of claim 12, wherein the articulation actuators comprise longitudinally translating camming actuators.

16. The surgical instrument of claim 12, wherein the articulation actuators comprise buckling members with positionable proximal ends.

17. A surgical instrument, comprising:
a proximal portion configured for manipulation external to a patient;
an elongate shaft attached to the proximal portion;
an end effector;
an articulation joint pivotally attaching the end effector to the elongate shaft;
a slide bar received for lateral movement in the elongate shaft and comprising a distal end engaged to pivot the end effector about the articulation joint and a locking mechanism moved into binding engagement with the elongate shaft in response to a backdriving force on the end effector.

18. The surgical instrument of claim 17, further comprising a gear segment attached to the end effector, the slide bar further comprising a distally attached rack engaged to the gear segment.

19. The surgical instrument of claim 17, wherein the locking mechanism comprises a channel formed laterally in the elongate shaft and a rib perpendicularly attached to the slide bar and received in the channel, the slide bar further comprising a flexible longitudinal portion connecting the rib to the distal end of the slide bar, wherein backdriving of the end effector bends the longitudinal portion, thereby cocking the rib into binding engagement in the channel.

20. The surgical instrument of claim 17, wherein the locking mechanism comprises engagement teeth laterally translating proximate to a lateral engagement surface attached to the elongate shaft, the surgical instrument further comprising a biasing member urging the slide bar and the engagement teeth distally out of engagement with the lateral engagement surface until the end effector is backdriven.
